# EUROPEAN PATENT SPECIFICATION

(11) **EP 4 230 209 B1**
(45) Date of publication and mention of the grant of the patent: **30.10.2024**
(21) Application number: 21894866.9
(22) Date of filing: 29.09.2021
(51) Int. Cl.: A61K 31/513, A61K 31/522, A61K 31/675, A61K 31/683, A61K 31/7072, A61K 45/06, A61K 39/12, A61P 31/20, A61K 31/685, A61K 39/00

(54) **PHARMACEUTICAL COMPOSITION, PHARMACEUTICAL COMBINED FORMULATION, AND COMBINED FORMULATION KIT FOR PREVENTION OR TREATMENT OF CHRONIC HEPATITIS B, EACH COMPRISING, AS ACTIVE INGREDIENT, ORAL ANTIVIRAL AGENT AND THERAPEUTIC VACCINE INCLUDING LIPOPEPTIDE AND POLY(I:C) ADJUVANT**
PHARMAZEUTISCHE ZUSAMMENSETZUNG, PHARMAZEUTISCHE KOMBINATION UND KOMBINATIONS-KIT ZUR PRÄVENTION ODER BEHANDLUNG VON CHRONISCHER HEPATITIS B
COMPOSITION PHARMACEUTIQUE, FORMULATION COMBINÉE PHARMACEUTIQUE ET KIT DE FORMULATION COMBINÉE POUR LA PRÉVENTION OU LE TRAITEMENT DE L'HÉPATITE B CHRONIQUE, COMPRENANT CHACUN, EN TANT QUE PRINCIPE ACTIF, UN AGENT ANTIVIRAL ORAL ET UN VACCIN THÉRAPEUTIQUE COMPRENANT UN LIPOPEPTIDE ET UN ADJUVANT POLY(I:C)

(30) Priority: 18.11.2020 KR 20200154793
(43) Date of publication of application: 23.08.2023
(73) Proprietor: Cha Vaccine Research Institute Co., Ltd, Seongnam-si, Gyeonggi-do 13230 (KR)
(72) Inventor: YUM, Jung Sun, Seongnam-si Gyeonggi-do 13588 (KR); AHN, Byung Cheol, Yongin-si Gyeonggi-do 16879 (KR); BAEK, Seung Hee, Seongnam-si Gyeonggi-do 13208 (KR); JEONG, Sookyung, Anyang-si Gyeonggi-do 14044 (KR)
(74) Representative: EP&C
(86) International application number: PCT/KR2021/013317
(87) International publication number: WO 2022/108094

(56) References cited:
- JP-A- 2018 534 304
- KR-A- 20020 020 748
- KR-A- 20170 132 327
- KR-B1- 100 900 837
- KR-B1- 101 501 583
- KR-B1- 102 292 147
- US-A1- 2012 082 720
- BONI CAROLINA ET AL: "Combined GS-4774 and Tenofovir Therapy Can Improve HBV-Specific T-Cell Responses in Patients With Chronic Hepatitis", GASTROENTEROLOGY, vol. 157, no. 1, 1 July 2019 (2019-07-01), US, pages 227 - 241.e7, XP093093713, ISSN: 0016-5085, DOI: 10.1053/j.gastro.2019.03.044

## Description

### BACKGROUND OF THE INVENTION

### 1. Field of the Invention

The present invention relates to a pharmaceutical composition, a pharmaceutical combined formulation, and a combined formulation kit for the prevention or treatment of chronic hepatitis B, each comprising an oral antiviral agent and a therapeutic vaccine including a lipopeptide and a poly(I:C) adjuvant as active ingredients.

### 2. Description of the Related Art

Currently, there are a total of 8 oral antiviral agents and 1 injection (peginterferon ex) as shown in Table 1 below for the treatment of chronic hepatitis B, and oral antiviral agents are divided into drugs with a high genetic barrier and drugs without, depending on the incidence of resistance.

**[Table 1]**

| Types of therapeutic agents for chronic hepatitis B | | | |
|---|---|---|---|
| **Classific ation (characte ristic)** | **Oral antiviral agent** | | **Injection** |
| | **High genetic barrier to resistance incidence (preferred)** | **Low genetic barrier to resistance incidence (not preferred)** | **Immunomodulat or** |
| **Name (componen t)** | 1. Entecavir | 1. Lamivudine | 1. Pegylatedinte rferon alfa 2a |
| | 2. Tenofovirdisopro xil fumarate (TDF) | 2. Telbivudine | |
| | | 3. Clevudine | |
| | | 4. Adefovirdipivox il (Adefovir) | |
| | 3. Tenofoviralafena mide fumarate (TAF) | | |
| | 4. Besifovirdipivox il maleate (Besifovir) | | |

Oral antiviral agents are nucleot(s)ide analogues that act on the process of converting pregenomic RNA of hepatitis B virus into DNA and interfere with normal DNA production, thereby inhibiting hepatitis B virus proliferation.

Peginterferon α, an injection, is known to have roles in destroying cccDNA and viral mRNA, inhibiting viral DNA replication, and regulating the immune response to virus-infected hepatocytes. However, the injection has a disadvantage in that the response rate for the therapeutic effect is low and that it cannot be used for people with reduced liver function due to the risk of liver failure. In addition, the use of peginterferon α should be considered carefully, as it can cause severe side effects such as depression. Combination therapy with peginterferon α and an oral antiviral agent has been suggested to be a more effective method for HBsAg loss, but the effect was not clear in genotype C, which accounts for the majority of Korean patients.

Therefore, domestic and international guidelines for chronic hepatitis B treatment recommend oral antiviral agents with a high genetic barrier to resistance as a first-line treatment, considering the advantages and disadvantages of each treatment.

Although oral antiviral agents can effectively inhibit the proliferation of hepatitis B virus, it is known that HBsAg loss, which is an index of a functional cure, rarely occurs in patients with chronic hepatitis B because they are not therapeutic agents that completely remove cccDNA in the nucleus.

When confirming the results of major clinical trials of oral antiviral agents, in the case of HBeAg-negative patients, there was no HBsAg loss after 1 year of treatment, as shown in Table 2 below (Non-patent reference 1, EASL 2017 Clinical Practice Guidelines on the management of hepatitis B virus infection).

**[Table 2]**

| Results of evaluating oral antiviral agent efficacy in HBeAg(-) patients | | | | | | |
|---|---|---|---|---|---|---|
| | **Nucleoside analogues** | | | **Nucleoside analogues** | | |
| | **LAM** | **TBV** | **ETV** | **ADV** | **TDF** | **TAF** |
| Dose | 100mg | 600mg | 0.5mg | 10mg | 245mg | 25mg |
| HBV DNA <60-80 IU/ml | 72∼73% | 88% | 90% | 51∼63% | 93% | 94% |
| ALT nomalisatioin | 71∼79% | 74% | 78% | 72∼77% | 76% | 83% |
| HBs Ag loss | 0% | 0% | 0% | 0% | 0% | 0% |

In the case of HBeAg-positive patients, the HBsAg loss rate after 1 year of treatment was only 3% (TDF) at most, as shown in Table 3 below.

**[Table 3]**

| Results of evaluating oral antiviral agent efficacy in HBeAg(+) patients | | | | | | |
|---|---|---|---|---|---|---|
| | **Nucleoside analogues** | | | **Nucleoside analogues** | | |
| | **LAM** | **TBV** | **ETV** | **ADV** | **TDF** | **TAF** |
| Dose | 100mg | 600mg | 0.5mg | 10mg | 245mg | 25mg |
| Anti-Hbe-seroconversion | 16∼18% | 22% | 21% | 12-∼18% | 21% | 10% |
| HBV DNA <60-80 IU/ml | 36∼44% | 60% | 67% | 13∼21% | 76% | 64% |
| ALT nomalisatioin | 41∼72% | 77% | 68% | 48∼54% | 68% | 72% |
| HBs Ag loss | 0∼1% | 0.5% | 2% | 0% | 3% | 1% |

HBeAg loss and seroconversion, and HBsAg loss (or reduction) and seroconversion rate, which are the efficacy evaluation items of oral antiviral agents known to date, are as follows. It can be seen that it is difficult to achieve HBsAg loss with oral antiviral agents through the following contents.

### (1) Phase 3 clinical trial results for Tenofoviralafenamide (TAF)

The results of the second year of the phase 3 clinical trial for TAF (nucleotide family), the most recently approved therapeutic agent for chronic hepatitis B, have been announced (Non-patent reference 2, Tenofoviralafenamide versus tenofovirdisoproxil fumarate for the treatment of patients with HBeAg-negative chronic hepatitis B virus infection: a randomised, double-blind, phase 3, non-inferiority trial (Lancet GastroenterolHepatol 2016; 1: 196-206); Non-patent reference 3, Tenofoviralafenamide versus tenofovirdisoproxil fumarate for the treatment of HBeAg-positive chronic hepatitis B virus infection: a randomised, double-blind, phase 3, non-inferiority trial (Lancet GastroenterolHepatol 2016; 1: 185-95); Non-patent reference 4, 96 weeks treatment of tenofoviralafenamide vs. tenofovirdisoproxil fumarate for hepatitis B virus infection (Journal of Hepatology, 2018)). The selection criteria for the phase 3 clinical trials of TAF included both patients with antiviral treatment experience and patients receiving treatment for the first time.

### 1-1. Summary of key efficacy results

**[Table 4]**

| Phase 3 clinical trial results for Tenofoviralafenamide (TAF) | | | | | | | | |
|---|---|---|---|---|---|---|---|---|
| | **Phase 3 clinical trial (1 year results)** | | | | **Phase 3 clinical trial (2 year results)** | | | |
| **Baselin e HBeAg** | **HBeAg(+)** | | **HBeAg(-)** | | **HBeAg(+)** | | **HBeAg(-)** | |
| **Group** | **TDF (N= 292)** | **TAF (N= 581)** | **TDF (N= 140)** | **TAF (N= 285)** | **TDF (N= 292)** | **TAF (N= 581)** | **TDF (N= 140)** | **TAF (N= 285)** |
| **HBeAg loss** n/N (%) | 34/28 5 (12%) | 78/56 5 (14%) | NA | NA | 51/28 5 (18%) | 123/ 565 (22% ) | NA | NA |
| **HBeAg serocon version** n/N (%) | 23/28 5 (8%) | 58/56 5 (10%) | NA | NA | 35/28 5 (12%) | 99/5 65 (18% ) | NA | NA |
| **HBsAg loss** n/N (%) | 1/288 (1%) | 4/576 (1%) | 0 | 0 | 4/288 (1%) | 7/57 6 (1%) | 0 | 1/28 1 (<1% ) |
| **HBsAg serocon version** n/N (%) | 0 | 3/576 (1%) | 0 | 0 | 0 | 6/57 6 (1%) | 0 | 1/28 1 (<1% ) |
| **Average reducti on of HBsAg** log₁₀IU/ ml | No results | | | | -0.64 | - 0.51 | - 0.10 | - 0.14 |

As shown in Table 4, the HBeAg loss rate was 14% at the first year and 22% at the second year in the TAF group, and 12% at the first year and 18% at the second year in the TDF group, which were lower than those in the TAF group. In addition, the HBsAg loss rate was very low at around 1% in both groups at both year 1 and year 2.

### (2) Phase 3 clinical trial results for Tenofovirdisoproxil fumarate (TDF) and efficacy results of long-term follow-up studies

In the case of TDF (nucleotide family), which has the largest share in the chronic hepatitis B treatment market, even the 10-year follow-up results of phase 3 clinical trials have been announced (Non-patent reference 5, TenofovirDisoproxil Fumarate versus AdefovirDipivoxil for Chronic Hepatitis B (N engl j med 359;23, 2008); Non-patent reference 6, Three-Year Efficacy and Safety of TenofovirDisoproxil Fumarate Treatment for Chronic Hepatitis B (GASTROENTEROLOGY 2011;140:132-143); Non-patent reference 7, Seven-Year Efficacy and Safety of Treatment with TenofovirDisoproxil Fumarate for Chronic Hepatitis B Virus Infection (Dig Dis Sci. 2015 May; 60(5):1457-64); Non-patent reference 8, Ten-year efficacy and safety of tenofovirdisoproxil fumarate treatment for chronic hepatitis B virus infection (Liver International. 2019;00:1-8.)). The main selection criteria for the phase 3 clinical trial were chronic hepatitis B patients who had been taking nucleot(s)ide-based antiviral drugs for less than 12 weeks. In the HBeAg-negative group, up to 120 out of 382 patients who had been taking Lamivudine or Emtricitabine (nucleoside family) for more than 12 weeks were allowed to enroll. In addition, HBeAg positive and HBeAg negative were classified and proceeded, and AdefovirDipivoxil (nucleotide family) was used as an active control group.

### 2-1. Summary of key efficacy results

**[Table 5]**

| Phase 3 clinical trial results for Tenofovirdisoproxil fumarate (TDF) and efficacy results of long-term follow-up studies | | | | | | | | |
|---|---|---|---|---|---|---|---|---|
| | **Phase 3 clinical trial (1 year results)** | | **3-year follow-up results (585 enrolled, 542 completed)** | | **7-year follow-up results (437 completed)** | | **10-year follow-up results (203 completed)** | |
| **Basekine HBeAg** | **HBeAg (+) (N=176)** | **HBeAg (-) (N=250)** | **HBeAg (+)** | **HBeAg (-)** | **HBeAg (+)** | **HBeAg (-)** | **HBeAg (+)** | **HBeAg (-)** |
| **HBeAg loss** n/N (%) | No result | NA | 34% | NA | 84/154 (54.5%) | NA | 12/23 (52.2%) | NA |
| **HBeAg seroconversion n/N (%)** | 32/153 (21%) | NA | 26% | NA | 61/154 (39.6%) | NA | 6/22 (27.3%) | NA |
| **HBsAg loss** n/N (%) | 5/158 (3.2%) (non-Asian) | 0 | 8% (n=20) [kaplan-meier] (non-Asian) | 0 | 11.8% (n=27) [kaplan-meier] | Fifth year: 1 | 4/81 (4.9%) (non-Asian) | 4/117 (3.4%) (non-Asian) |
| **HBsAg seroconversion** n/N (%) | 2/158 (1.3%) (non-Asian) | 0 | 6% (n=15) [kaplan-meier] (non-Asian) | 0 | 9.7% (n=21) [kaplan-meier] | 0 | No result | |

As shown in Table 5, for the HBeAg loss rate, there was no mention of the result at the first year, and the rate was 34% at the third year, 54.5% at the seventh year, and 52.2% at the tenth year. The HBeAg seroconversion rate was 21% (TDF group) at the first year, 26% at the third year, 39.6% at the seventh year, and 27.3% at the tenth year, respectively. The HBsAg loss rate in the HBeAg-positive group was 3.2% at the first year (TDF group), 8% at the third year (Kaplan-Meier analysis), 11.8% at the seventh year (Kaplan-Meier analysis), and 4.9% at the tenth year. In the HBeAg-negative group, only one patient had HBsAg loss until the fifth year, and the HBsAg loss rate was 3.4% at the tenth year. Above all, it is worth noting that the loss of HBsAg did not appear in Asians (There was no mention of race in the results at the seventh year, but there were no Asians with HBsAg loss in the results at the tenth year.). Kaplan-meier analysis, which was used to analyze HBsAg loss at the third and seventh years, is a method used for survival analysis in anticancer drug clinical trials to estimate the probability of survival over a period of time using death outcomes during the observation period. During the long-term follow-up study, patients with HBsAg loss and seroconversion dropped out or discontinued antiviral treatment, resulting in a loss of HBsAg loss subjects at the time of efficacy evaluation. To correct for this, Kaplan-meier analysis was used for follow-up study results at the third and seventh year of the TDF trial.

### 2-2. Detailed efficacy results by year

### a. Results of phase 3 clinical trial (1 year):

As a result of the efficacy evaluation at the first year, 21% of patients in the HBeAg-positive group had HBeAg seroconversion, 3.2% of patients had HBsAg loss, and 1.3% of patients had HBsAg seroconversion.

### b. Results of 3-year follow-up:

Among the subjects who completed the phase 3 clinical trial, 585 patients participated in the long-term follow-up study, and 542 patients completed the 3-year follow-up study. In addition, the patients assigned to the active control group also participated in the study by replacing the antiviral agent with TDF. As a result of evaluating the efficacy of the 3-year follow-up study, 34% of patients in the HBeAg-positive group showed HBeAg loss.

HBsAg loss occurred in 20 patients over 3 years, and all of them were HBeAg positive group subjects and non-Asians. Of these, 14 stopped taking antiviral agents.

### c. Results of 7-year follow-up:

Seven-year follow-up was completed for a total of 437 patients. The HBeAg loss rate was 54.5%, and 28 patients showed HBsAg loss over 7 years, and 25 of them stopped taking antiviral agents. Only 1 of 28 patients belonged to the HBeAg-negative group.

### d. Results of 10-year follow-up:

Ten-year follow-up was completed for a total of 203 patients. The HBeAg seroanalysis results could be confirmed in only 23 patients, and as a result, HBeAg loss was found in 52.2% of patients. In addition, HBsAg loss was also observed in 4.0% (8/198) of patients.

### 2-3. Tendency for HBsAg levels to decrease

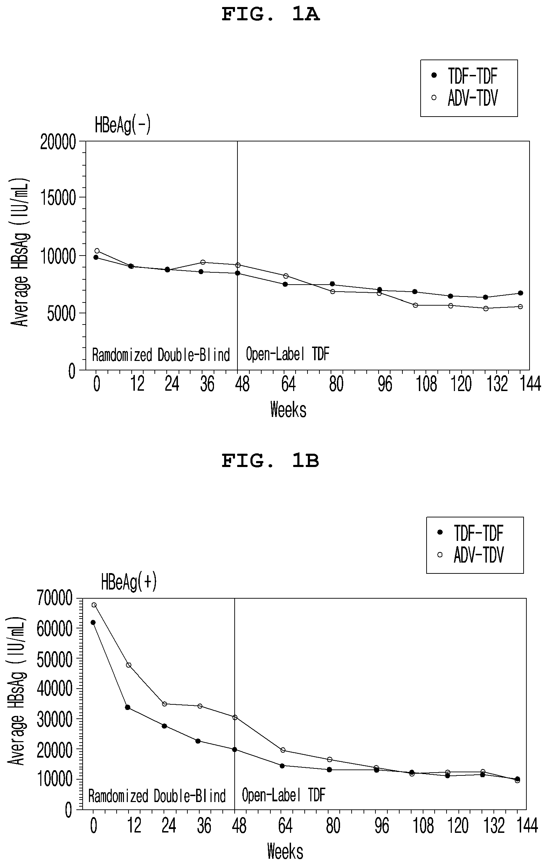

Furthermore, the results of the TDF phase 3 clinical trial confirmed that not only HBsAg loss, but also reduction of HBsAg quantitative levels is not easy. In the TDF phase 3 clinical trial, patients who started receiving oral antiviral agent treatment for the first time showed a slight decrease in HBsAg levels at the beginning of treatment, but the degree and speed of the decrease slowed after 1 year of taking the antiviral agent, and after 2 years (96 weeks), there has been no significant change since then (see Figure 1). This is a result confirming that long-term administration of oral antiviral agents does not cause a continuous reduction of HBsAg.

### (3) Phase 3 clinical trial results for Entecavir and efficacy results of long-term follow-up studies

In the case of TDF (nucleotide family), which has the largest share in the chronic hepatitis B treatment market, even the 10-year follow-up results of phase 3 clinical trials have been announced

The results of phase 3 clinical trials for Entecavir, which had the No. 1 market share before TDF development, were announced up to the 5-year follow-up results (Non-patent reference 9, Entecavir Therapy for up to 96 Weeks in Patients With HBeAg-Positive Chronic Hepatitis B (GASTROENTEROLOGY 2007;133:1437-1444); Non-patent reference 10, Entecavir Treatment for up to 5 Years in Patients with Hepatitis B e Antigen-Positive Chronic Hepatitis B (HEPATOLOGY, Vol. 51, No. 2, 2010)). The phase 3 clinical trial for Entecavir was conducted on patients who had no experience taking nucleosidebased antiviral agents. Like the clinical trials for other antiviral drugs, the clinical trials were conducted by dividing HBeAg positive and HBeAg negative, but in the case of the HBeAg negative group, the results of HBeAg loss or HBsAg loss were rarely mentioned in the papers.

**[Table 6]**

| Phase 3 clinical trial results for Entecavir and efficacy results of long-term follow-up studies | | |
|---|---|---|
| | **Phase 3 clinical trial (2 year results)** | **5 year results** |
| **Baseline HBeAg** | **HBeAg(+)** | |
| **HBeAg loss** n/N (%) | No result | |
| **HBeAgseroconversion** n/N (%) | 110/354 (31%) | 33/141 (23%) |
| **HBsAg loss** n/N (%) | 18/354 (5%) | 2/145 (1.4%) |
| **HBsAgseroconversion** n/N (%) | 6/354 (2%) | No result |

As shown in Table 6, the HBeAg seroconversion rate was 31% at the second year, and the seroconversion subjects did not participate in the 5-year long-term follow-up study because they corresponded to the treatment response in the protocol. The HBeAg seroconversion rate in the 5-year follow-up was 23%. The HBsAg loss rate was 5% at the second year and 1.4% at the fifth year. In the case of Entecavir, apart from the phase 3 clinical trial, a seven-year real-world cohort study was conducted in Hong Kong for Chinese. As a result, the HBeAg seroconversion rate for 7 years analyzed by Kaplan-meier analysis was 82.1%. HBsAg loss was seen in only 4 of 160 (2.5%) patients (Non-patent reference 11, Seven-Year Treatment Outcome of Entecavir in a Real-World Cohort: Effects on Clinical Parameters, HBsAg and HBcrAg Levels (Clinical and Translational Gastroenterology, 2017)).

### (4) A review of clinical trial results for oral antiviral agents: Limitations of monotherapy

Through the summary of major clinical trial results for the three most commonly used drugs as the first-line treatment of chronic hepatitis B, it was confirmed once again that HBsAg loss, which means a functional cure for chronic hepatitis B, is difficult to occur with oral antiviral agents. Patients with chronic hepatitis B, who are difficult to cure, must take oral antiviral agents for almost the rest of their lives to suppress the virus. If these patients stop taking antiviral agents without being completely cured, the hepatitis B virus proliferation becomes active again, so treatment cannot be stopped in the middle.

However, long-term use of antiviral agents causes other problems such as resistance development, or occurrence of side effects like abnormal renal function or bone metabolic disease, so patients with chronic hepatitis B are in difficulties under any circumstances. Therefore, it is necessary to develop an effective therapeutic agent that can rapidly lead to a complete cure of chronic hepatitis B.

Accordingly, the present inventors have performed a highly inventive effort for the purpose of completely curing chronic hepatitis B, which is currently impossible to cure. As a result, the inventors completed the present invention by confirming in clinical trials that co-administration of an oral antiviral agent and a therapeutic vaccine comprising a lipopeptide and a poly(I:C) adjuvant resulted in a significant escalation effect (i.e., a synergistic effect) in the treatment index of chronic hepatitis B in patients receiving the standard treatment of antiviral agents, compared to patients receiving the standard treatment of antiviral agents alone, resulting in a cure.

### SUMMARY OF THE INVENTION

It is an object of the present invention to provide a pharmaceutical composition for the prevention or treatment of chronic hepatitis B that enables a complete cure due to a significant escalation effect (i.e., a synergistic effect) in the treatment index of chronic hepatitis B, a pharmaceutical combined formulation, a combined formulation kit, and a method for generating an immune response against chronic hepatitis B in a subject comprising a step of administering the same to the subject.

To achieve the above object, in an aspect of the present invention, the present invention provides a pharmaceutical combined formulation for the prevention or treatment of chronic hepatitis B comprising an antiviral agent for hepatitis B; and a therapeutic vaccine composition including an antigen, a lipopeptide and a poly(I:C) adjuvant.

In another aspect of the present invention, the present invention provides a pharmaceutical composition for the prevention or treatment of chronic hepatitis B comprising an antiviral agent for hepatitis B; and a therapeutic vaccine composition including an antigen, a lipopeptide and a poly(I:C) adjuvant.

In another aspect of the present invention, the present invention provides a method for generating an immune response against chronic hepatitis B in a subject comprising a step of administering a pharmaceutical combined formulation for the prevention or treatment of chronic hepatitis B comprising an antiviral agent for hepatitis B; and a therapeutic vaccine composition including an antigen, a lipopeptide and a poly(I:C) adjuvant to the subject.

In another aspect of the present invention, the present invention provides a combined formulation kit for the prevention or treatment of chronic hepatitis B comprising a first component containing an antiviral agent for hepatitis B; and a second component containing a vaccine composition including an antigen, a lipopeptide and a poly(I:C) adjuvant.

### ADVANTAGEOUS EFFECT

When the pharmaceutical composition, the pharmaceutical combined formulation, and the combined formulation kit provided in one aspect of the present invention are administered/used in hepatitis B patients, a remarkable synergy occurs in terms of therapeutic index for chronic hepatitis B, compared to patients who have undergone standard therapy including the administration of conventional antiviral agents, exhibiting the possibility of completely curing the disease.

### BRIEF DESCRIPTION OF THE DRAWINGS

Figure 1a is a graph showing the changes in HBsAg levels (3-year results) in TDF phase 3 clinical trials for HBsAg(-) patients. TDF-TDF: TDF test group, taking TDF in follow-up study; ADV-TDF: ADF test group, taking TDF in follow-up study.
Figure 1b is a graph showing the changes in HBsAg levels (3-year results) in TDF phase 3 clinical trials for HBsAg(+) patients. TDF-TDF: TDF test group, taking TDF in follow-up study; ADV-TDF: ADF test group, taking TDF in follow-up study.
Figure 2 is a diagram showing the importance of T cell activation in chronic hepatitis B patients.
Figure 3 is a diagram showing the results of analyzing the 1/2a effectiveness of CVI-HBV-002.
Figure 4 is a graph showing the results of comparing the HBeAg loss rate in the CVI-HBV-002 1/2a clinical trial and the TAF phase 3 clinical trial.
Figure 5 is a graph confirming the maintenance of T cell activation after administration of CVI-HBV-002.
Figure 6 is a graph showing the results of comparing the HBeAg loss rate in the CVI-HBV-002 long-term follow-up study and the TAF phase 3 clinical trial.

### DESCRIPTION OF THE PREFERRED EMBODIMENTS

Hereinafter, the present invention is described in detail.

The embodiments of this invention can be modified in various other forms, and the scope of the present invention is not limited to the embodiments described below. It is well understood by those in the art who has the average knowledge on this field that the embodiments of the present invention are given to explain the present invention more precisely.

In addition, the "inclusion" of an element throughout the specification does not exclude other elements, but may include other elements, unless specifically stated otherwise.

In an aspect of the present invention, the present invention provides a pharmaceutical combined formulation for the prevention or treatment of chronic hepatitis B comprising an antiviral agent for hepatitis B; and a vaccine composition including an antigen, a lipopeptide and a poly(I:C) adjuvant.

At this time, the antiviral agent for hepatitis B can be an oral antiviral agent, and as a specific example of the oral antiviral agent, Entecavir, Tenofovirdisoproxil fumarate (TDF), Tenofoviralafenamide fumarate (TAF), Besifovirdipivoxil maleate, Lamivudine, Telbivudine, Clevudine, Adefovirdipivoxil, and the like can be used alone or in combination of two or more without limitation.

Hereinafter, the development trend of a new chronic hepatitis B treatment is described.

Since it is difficult to cure (functional cure) chronic hepatitis B with existing oral antiviral agents, the development of a new chronic hepatitis B treatment is actively underway. Recently, direct-acting antiviral agents with a new mechanism have been developed, and the representative types include RNA interferences that inhibit protein synthesis by binding to viral mRNA and breaking it down into small units, or capsid inhibitors that prevent normal production of a capsid covering genetic materials. On the other hand, immunotherapeutic agents of strategies with strategies to reduce HBsAg by activating weakened immune cells of patients are being developed, and the representative types include cytokines, tol-like receptor ligands that activate innate immunity, and therapeutic vaccines that control adaptive immunity by administering antigens. Development trends and types of chronic hepatitis B therapeutic agents are shown in Table 7 in the specification of the present invention.

Regarding the current status of clinical trials of newly developed chronic hepatitis B treatments, almost all clinical trials are being conducted with a design that confirms the treatment effect of the combination administration of clinical trial drugs while continuing to take antiviral agents, which are standard treatments. This is to protect patients by combining standard treatment, and because it was confirmed that it is difficult to completely cure chronic hepatitis B with only one type of treatment through clinical trials of oral antiviral agents or combined administration studies of oral antiviral agents and interferon. The development phase of chronic hepatitis B treatment and whether or not to administrate antiviral agents in combination are summarized in Table 7 below.

**[Table 7]**

| Development phase of chronic hepatitis B treatment and whether or not to administrate antiviral agents in combination | | | | |
|---|---|---|---|---|
| **Compound** | | **Sponsor** | **Phase of development** | **Combined administration of antiviral agents (types)** |
| HBV entry inhibitors | | | | |
| | Myrcludex B | MyrPharmaceuticals | Phase I/II | O (Tenofovir) |

| Inhibition of gene expression / gene silencing | | | | |
|---|---|---|---|---|
| | GSK3389404 | GlaxoSmith Kline | Phase II | O (nucleos (t) ide analogue) |
| | JNJ-3989 (ARO-HBV) | Janssen | Phase I/II | O (nucleos (t) ide analogue) |
| | BRII-835 | Brii Bioscience | Phase II | O (nucleos (t) ide analogue) |

| Core protein (Capsid) assembly modulators (CpAMs) | | | | |
|---|---|---|---|---|
| | ABI-H0731 | Assembly Bioscience | Phase IIa | O (Entecavir) |
| | ABI-H2158 | Assembly Bioscience | Phase II | O (Entecavir) |
| | RO7049389 | Roche | Phase II | O (nucleos (t) ide analogue) |
| | JNJ-56136379 | Janssen | Phase II | O (Tenofovir) |
| | ABI-H2158 | Assembly Bioscience | Phase I | O (Entecavir) |
| | GLS4JHS | Jilin University | Phase II | O (Entecavir) |

| HBsAg release inhibitors | | | | |
|---|---|---|---|---|
| | Nucleic acid polymers (REP compound series) | Replicor | Phase II | O (nucleos (t) ide analogue) |

| Targeting cell intrinsic and Innate Immune responses | | | | |
|---|---|---|---|---|
| | RO7020331 TLR 7 agonist | Roche | Phase I | O (nucleos (t) ide analogue) |
| | GS-9620 TLR 7 agonist | Gilead | Phase II | O (TDF) |
| | GS-9688 TLR 8 agonist | Gilead | Phase I | O (nucleos (t) ide analogue) |
| | AIC649 TLR 9 agonist | AiCuris | Phase I | Unknown |
| | InarigivirsoproxilRIG-I agonist | Spring Bank | Phase II | O (TAF) |

| Targeting adaptive immune responses | | | | |
|---|---|---|---|---|
| | CVI-HBV-002 | Cha Vaccine Institute | Phase IIb | O (Tenofovir) |
| | TG1050/T101 | Transgene/Talsy | Phase I | O (TDF or Ente cavi r) |
| | BRII-179 | Brii Bioscience | Phase Ib/IIa | O (nucleos (t) ide analogue) |
| | VTP-300 | Vaccitech | Phase Ib/IIa | O (Entecavir, Tenofovir, Besi fovir) |
| | HepTcell(FP-02.2) | Altimmune | Phase I | O (Entecavir or Tenofovir) |
| | JNJ-64300535 | Janssen | Phase I | O (nucleos (t) ide analogue) |
| | INO-1800 | Inovio | Phase I | O (nucleos (t) ide analogue) |

| Immune checkpoint inhibitors | | | | |
|---|---|---|---|---|
| | Nivolumab | Bristol-Myers Squib | Phase I | O (nucleos (t) ide analogue) |
| | HLX10 | Henlix | Phase II | O (nucleos (t) ide analogue) |
| | ASC22 | Ascletis Pharmaceuticals | Phase II | O (nucleos (t) ide analogue) |

On the other hand, since the combied formulation is for the prevention or treatment of chronic hepatitis B, the antigen is preferably the entire surface antigen (L-HBsAg) of HBV (Hepatitis B Virus).

In addition, the adjuvant is a substance or a combination of substances that increases or induces an immune response to a vaccine antigen in a desirable direction in order to enhance the clinical effect of a vaccine when used together with a vaccine antigen. The main function of an adjuvant is to enhance and improve the clinical effectiveness of a vaccine, such as increasing and regulating an immune response to a vaccine antigen or extending the duration of the protective effect by acting on direct or indirect immune stimulation and antigen delivery. When a pathogenic bacterum or virus is infected, the surface receptor (pattern recognition receptor) of an immune cell recognizes the unique pattern (pathogen-associated molecular pattern, PAMP) of a pathogenic microorganism and causes an innate immune response. Toll like receptors (TLRs) are representative surface receptors, and about 13 types are known in humans. TLR ligands, which respond to tall-like receptors, are being developed as adjuvants because they directly stimulate immune cells to activate innate immune responses and induce humoral immunity and cellular immune responses, the acquired immunity against vaccine antigens, to protect the human body from infectious agents or contribute to tissue recovery.

Lipopeptide, a specific example of the adjuvant, was first synthesized by J. Metzger et al as a synthetic analogue of a lipopeptide derived from bacteria and mycoplasma (Metzger, J. et al., 1991, Synthesis of novel immunologically active tripalmitoyl-S-glycerylcysteinyllipopeptides as useful intermediates for immunogen preparations. Int. J. Peptide Protein Res. 37: 46-57). The molecular structure of the compound represented by the following formula (1) is N-palmitoyl-S-[2,3-bis(palmitoyloxy)-(2RS)-propyl]-[R]-cystein-SKKKK (pam3Cys-SKKKK), and various other analogues have been synthesized.

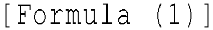

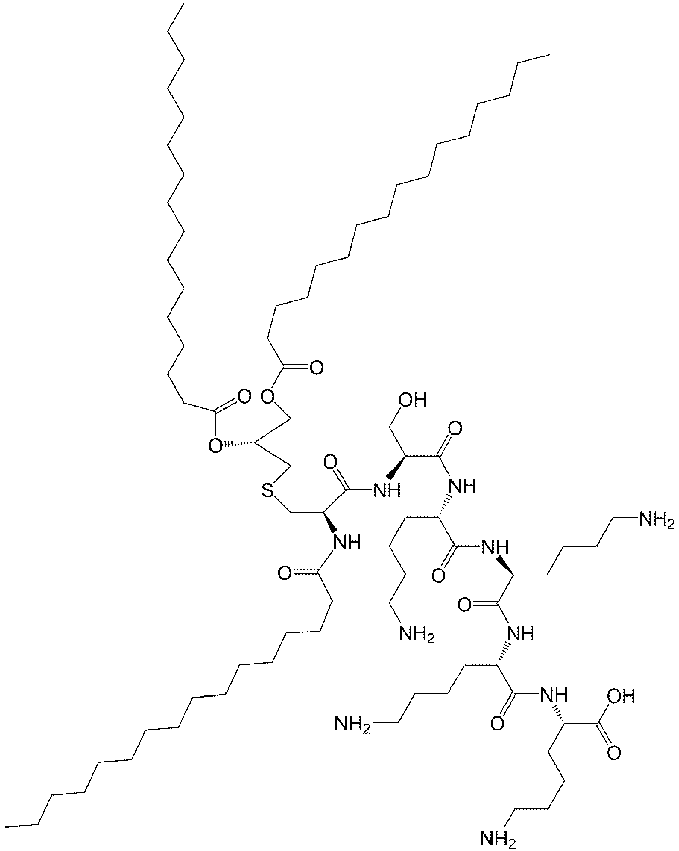

According to H. Schild et al., when Pam3Cys-Ser-Ser was combined with an influenza virus T cell epitope and administered to mice, virus-specific cytotoxic T lymphocytes (CTLs) were induced. In general, lipopeptides are known as ligands for TLR2. The use of such lipopeptides is not limited to Pam3Cys-SKKKK, and a lipopeptide can consist of a fatty acid bound to a glycerol molecule and several amino acids. Specific examples thereof include PHC-SKKKK, Ole2PamCys-SKKKK, Pam2Cys-SKKKK, PamCys(Pam)-SKKKK, Ole2Cys-SKKKK, Myr2Cys-SKKKK, PamDhc-SKKKK, PamCSKKKK, Dhc-SKKKK, and the like. The number of fatty acids in a molecule can be one or more. The number of amino acids in a lipopeptide can be one or more. In addition, the fatty acid and amino acid can be chemically modified. Furthermore, the lipopeptide can be a lipoprotein, either as a part of a molecule or as a whole molecule, derived from a gram-positive or gram-negative bacteria or mycoplasma.

In addition, the poly(I:C) has been used as a potent inducer of type 1 interferon in *in vitro* and *in vivo* studies. Moreover, poly(I:C) is known to stably and maturely form dendritic cells, the most potent antigen-presenting cells in mammals (Rous, R. et al 2004. poly(I:C) used for human dendritic cell maturation preserves their ability to secondarily secrete bioactive I1-12, International Immunol. 16: 767-773). According to these previous reports, poly(I:C) is a potent IL-12 inducer, and IL-12 is an important cytokine that induces cellular immune response and formation of IgG2a or IgG2b antibody by driving the immune response to develop Th1. In addition, poly(I:C) is known to have strong adjuvant activity against peptide antigens (Cui, Z. and F. Qui. 2005. Synthetic double stranded RNA poly I:C as a potent peptide vaccine adjuvant: Therapeutic activity against human cervical cancer in a rodent model. Cancer Immunol. Immunotherapy 16: 1-13). The poly (I:C) can have a length in a range of 50 to 5,000 bp, preferably 50 to 2,000 bp, and more preferably 100 to 500 bp, but not always limited thereto.

The lipopeptide and poly(I:C) can be included in the vaccine composition at a weight ratio of 0.1 to 10:1, a weight ratio of 1.25 to 2:1, a weight ratio of 1.25 to 1.5:1, or a weight ratio of 1.25:1, but not always limited thereto. However, the ratio can be adjusted to an appropriate level according to the patient's condition. In addition, the vaccine composition can be an aqueous solution formulation.

The vaccine composition can further include at least one selected from the group consisting of pharmaceutically acceptable carriers, diluents and adjuvants. For example, the vaccine composition can include a pharmaceutically acceptable carrier, and can be formulated for human or veterinary use and administered through various routes. The vaccine composition may be administered through oral, intraperitoneal, intravenous, intramuscular, subcutaneous, and intradermal routes. Preferably, it is formulated and administered as an injection. Injections can be prepared using aqueous solvents such as physiological saline and Ringer's solution, vegetable oils, higher fatty acid esters (e.g., ethyl oleate, etc.), and non-aqueous solvents such as alcohols (e.g., ethanol, benzyl alcohol, propylene glycol, glycerin, etc.) and can include pharmaceutical carriers such as stabilizers (e.g., ascorbic acid, sodium sulfite, sodium pyrosulfate, BHA, tocopherol, EDTA, etc.) to prevent deterioration, emulsifiers, buffers for pH control, preservatives for preventing microbial development (e.g., chimerosal, benzalkonium chloride, phenol, cresol, benzyl alcohol, etc.) and preservatives (e.g., phenylmercuric nitrate, thimerosal, benzalkonium chloride, phenol, cresol, benzyl alcohol, etc.) to inhibit microbial growth. The vaccine composition can be administered in a pharmaceutically effective amount.

At this time, the term "pharmaceutically effective amount" means an amount sufficient to exhibit a vaccine effect but an amount not to cause side effects or serious or excessive immune responses. The exact dosage concentration depends on the antigen to be administered, and can be easily determined by those skilled in the art according to factors well known in the medical field, such as the patient's age, weight, health, gender, sensitivity to drugs, administration route, and administration method. The composition of the present invention can be administered once or several times. In addition, the adjuvant can be further included in the vaccine composition as other known adjuvants other than lipopeptide and poly(I:C).

In another aspect of the present invention, the present invention provides a pharmaceutical composition for the prevention or treatment of chronic hepatitis B comprising an antiviral agent for hepatitis B; and a vaccine composition including an antigen, a lipopeptide and a poly(I:C) adjuvant.

In another aspect of the present invention, the present invention provides a method for generating an immune response against chronic hepatitis B in a subject comprising a step of administering a pharmaceutical combined formulation for the prevention or treatment of chronic hepatitis B comprising an antiviral agent for hepatitis B; and a vaccine composition including an antigen, a lipopeptide and a poly(I:C) adjuvant to the subject.

If the vaccine composition is administered to a human (patient), it can be administered in an amount effective to stimulate an immune response in vivo, for example, it can be administered to humans once or several times, and the dosage is 1-250 *µ*g*,* more preferably 10-100 *µ*g, but not always limited thereto.

In another aspect of the present invention, the present invention provides a combined formulation kit for the prevention or treatment of chronic hepatitis B comprising a first component containing an antiviral agent for hepatitis B; and a second component containing a vaccine composition including an antigen, a lipopeptide and a poly(I:C) adjuvant.

In another aspect of the present invention, the present invention provides a method for preventing, ameliorating or treating chronic hepatitis B comprising a step of administering an antiviral agent for hepatitis B; and a vaccine composition including an antigen, a lipopeptide and a poly(I:C) adjuvant to a subject.

In another aspect of the present invention, the present invention provides a use of [an antiviral agent for hepatitis B; and a vaccine composition including an antigen, a lipopeptide and a poly(I:C) adjuvant] for the preparation of a medicament for preventing, ameliorating or treating chronic hepatitis B.

When the pharmaceutical composition, the pharmaceutical combined formulation, and the combined formulation kit provided in one aspect of the present invention are administered/used in hepatitis B patients, a remarkable synergy occurs in terms of therapeutic index for chronic hepatitis B, compared to patients who have undergone standard therapy including the administration of conventional antiviral agents, exhibiting the possibility of completely curing the disease. This is directly supported by clinical trials of examples and experimental examples described below.

Hereinafter, the present invention will be described in detail by the following preparative examples, examples and experimental examples.

However, the following preparative examples, examples and experimental examples are only for illustrating the present invention, and the contents of the present invention are not limited thereto.

### Preparation of vaccine composition comprising antigen, lipopeptide and poly(I:C) adjuvant (CVI-HBV-002 therapeutic vaccine)

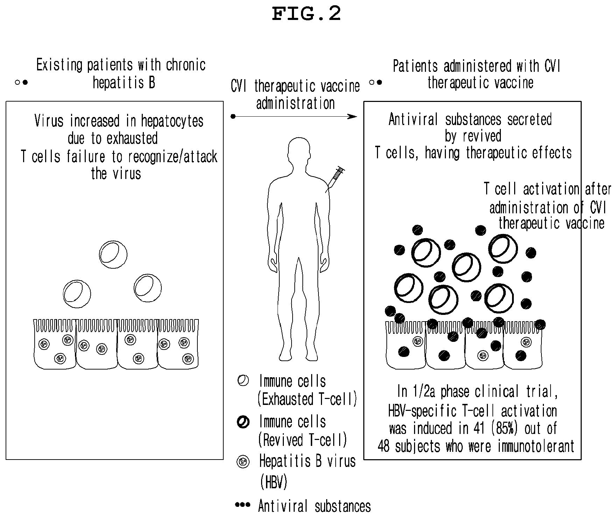

Recently, there is a growing need for the development of immunotherapeutic agents that can cause virus reduction in the body by resolving the lowered immune tolerance of patients with chronic hepatitis B. The therapeutic vaccine CVI-HBV-002 is composed of L-HBsAg, a third-generation antigen with excellent immunogenicity, and L-pampo [Pam3Cys-SKKKK + poly(I:C)], a powerful adjuvant. When this is administered in combination with an antiviral agent, a synergistic effect of suppressing hepatitis B virus proliferation, resolving immune tolerance in patients and activating T cells can be expected. The ultimate goal of developing CVI-HBV-002 is to induce adaptive immunity against hepatitis B virus, leading to a rapid cure (see Figure 2). More specifically, 200 or 400 *µ*g of poly(I:C) was added to 20 or 40 *µ*g of total surface antigen (L-HBsAg), mixed well, and then 250 or 500 *µ*g of Pam3Cys-SKKKK, a lipopeptide, was added to the mixture to prepare the theapeutic vacine CVI-HBV-002.

### Combined administration to hepatitis B patients who have undergone standard therapy

A clinical trial was conducted in which an oral antiviral agent and the therapeutic vaccine CVI-HBV-002 prepared above were co-administered to chronic hepatitis B patients who have undergone standard therapy including the administration of antiviral agents. In each experimental example described below, specific details such as the number of actually recruited patients, gender, age, and the type and dose of antiviral agents that have been prescribed are described. On the other hand, unless otherwise stated, patients with chronic hepatitis B were given antiviral agents through oral administration once a day, and the therapeutic vaccine CVI-HBV-002 prepared above was administered by intramuscular injection three or six times every four weeks, or six times every two weeks together with an oral antiviral agent from the start of the combined administration in experimental examples described below.

### Results of 1/2a clinical trial

### 1-1. Results of 1/2a clinical trial efficacy

The safety and tolerability of the therapeutic vaccine CVI-HBV-002 for hepatitis B was confirmed through a 1/2a-phase clinical trial in which an oral antiviral agent and the therapeutic vaccine CVI-HBV-002 prepared above were co-administered to chronic hepatitis B patients who have undergone standard therapy including the administration of conventional antiviral agents.

A total of 53 patients were recruited for the 1/2a clinical trial, of which 51 patients, excluding 2 patients who dropped out, were included in the efficacy evaluation analysis. Among them, 35 were male (68.6%) and 16 were female (31.4%), and their average age was 46 years old. The average length of time subjects took oral antiviral agents was approximately 70 months. As for the types of oral antiviral agents being taken, 21 patients took TDF alone, 14 patients took Entecavir alone, and 11 patients took Adefovir and other types of oral antiviral agents (Entecavir, Lamivudine or Telbivudine) together. Other than that, there were one patient taking TDF and Entecavir, one patient taking TDF and Telbivudine, one patient taking Lamivudine, one patient taking Telbivudine, and one patient taking three oral antiviral agents (Clevudine, Adefovir and TDF) together. They were orally administered once a day at a dose determined according to each antiviral agent they were taking (eq. Entecavir 0.5 mg, TDF 300 mg, Adefovir 10 mg).

The hepatitis B therapeutic vaccine CVI-HBV-002 was administered with an antigen dose of 20 ug or 40 ug 3 times every 4 weeks, 6 times every 4 weeks, or 6 times every 2 weeks, depending on the group to which the subjects were assigned.

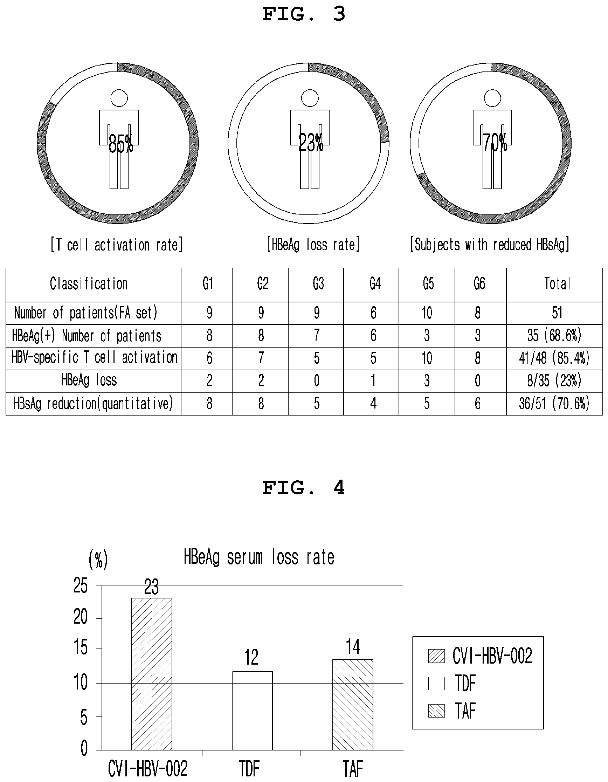

The results are shown in Figure 3.

Figure 3 is a diagram showing the results of analyzing the 1/2a effectiveness of CVI-HBV-002.

As shown in Figure 3, it was confirmed that HBV-specific T cell immune response was induced in 41 of 48 patients (85.4%) after vaccine administration, resulting in overcoming immune tolerance by vaccine administration. In addition, a very encouraging result was obtained in which HBeAg serum loss was induced in 8 out of 35 (23%) subjects who maintained HBeAg-positive status even after taking antiviral drugs for a long time, and 36 out of 51 patients (70.6%) showed a quantitative decrease in serum HBsAg. In Figure 3, the results mentioned above are highlighted in red. From these results, it can be confirmed that HBV-specific T cells were activated by the administration of CVI-HBV-002, which greatly affected the quantitative reduction of HBsAg and the loss of HBeAg.

### 1-2. Comparison of results with clinical trial control for Gilead therapetic vaccine (GS4774)

In order to compensate for the absence of a control group that took only antiviral agents in the 1/2a clinical trial, the control group results of the phase 2 clinical trial of Gilead's therapeutic vaccine (GS4774) were compared (Randomized phase II study of GS-4774 as a therapeutic vaccine in virally suppressed patients with chronic hepatitis B (Journal of Hepatology 2016 vol. 65 509-516)).

The results are shown in Tables 8 and 9 below.

**[Table 8]**

| Comparison of clinical trial results with competing products | | | |
|---|---|---|---|
| Company | GILEAD | | CHA |
| Clinical trial | Phase I (NCT0.779505) | Phase II (NCT01943799) | Phase I/II (NCT02693652) |
| Name | GS-4774 | GS-4774 | CVI-HBV-002 |
| Subject | Healthy subjects (n=60) | Chronic hepatitis B patients (n=178) | Chronic hepatitis B patients (n=53) |
| | | Antiviral agent users (>1yr) | Antiviral agent users |
| Usage/dosa ge | · 10 YU (n=20) | · antiviral agent alone (n=25) | · antiviral agent + CVI-HBV-002 |
| | · 40 YU (n=20) | | |
| | · 80 YU (n=20) | | |
| | | · antiviral agent + GS-4774; 2, 10, 40 YU (n=50/dose) | · antigen dosage: 20 ug or 40 ug |
| | | | · administrati on frequency: 3 times or 6 times |
| Administra tion schedule | Subcutaneous injections weekly or monthly | Subcutaneous injections at weeks 0, 4, 8, 12, 16 and 20 (6 times) | Intramuscular injections every 2 or 4 weeks |
| Primary endpoint | Safety | Changes in HBsAg levels (24 weeks) | Safety |
| Secondary endpoint | Immunogenicity of GS-4774 | · HBsAg loss & seroconversion | · HBeAg loss & seroconversio n |
| | | · HBeAg loss & seroconversion | |
| | | | · HBsAg loss & seroconversio n |
| | | · HBsAg 1log₁₀ reduction subjects | |
| | | | · HBV-specific T cell activation |
| | | | · HBV DNA level |
| Results | Safety cinfirmed | No significant decrease in serum HBsAg levels | Safety cinfirmed |
| | HBV specific T cell induction subjects: 51% (ELISPOT analysis) | | HBeAg loss subjects: 23% |
| | | HBsAg loss subjects: 11% | HBsAg loss subjects: 70.6% |
| | | HBsAg reduction in high-dose 40 YU group | |
| | | | HBV-specific T cell induction subjects: 85% (ELISPOT analysis) |

As shown in Table 8, Gilead's phase 2 clinical trial failed because it did not show a significant difference from the control group in the HBsAg change rate suggested as the primary endpoint, and the HBeAg loss rate in the GS4774 administration group was only 11%, which was significantly different from the HBeAg loss rate of 23% in the CVI-HBV-002 1/2a clinical trial.

**[Table 9]**

| Comparison with control group results of phase 2 clinical trial of GS4774 | | | |
|---|---|---|---|
| | | **Phase 2 clinical trial for GS4774 (control group)** | **1/2a clinical trial for CVI-HBV-002** |
| **Drug** | | **oral antiviral agent(N=27)** | **CVI-HBV-002 (N=51)** |
| **HBeAg loss,** n/N (%) | | 0 | 8/35 (23%) |
| **HBeAg seroconversion,** n/N (%) | | 0 | 2/35 (5.7%) |
| **Average reduction of HBsAg** log₁₀IU/ml | Evaluation point: Week12 | n=21 | n=17 (3 times every 4 weeks) |
| | | -0.004 (-0.041 to 0.033) | -0.021 (-0.094 to 0.023) |
| | Evaluation point: Week24 | n=21 | n=15 (6 times every 4 weeks) |
| | | -0.019 (-0.070 to 0.031) | -0.030 (-0.126 to 0.399) |

As shown in Table 9, as a result of comparison with the results of the CVI-HBV-002 clinical trial using the results of the control group of the phase 2 clinical trial of GS4774, the control group of the phase 2 clinical trial of GS4774 did not show HBeAg loss in the 1-year evaluation, which was a significant difference from the HBeAg loss rate of 23% in the CVI-HBV-002 1/2a clinical trial. In addition, in the case of HBsAg reduction, comparison was made using the 1/2a results analyzed at the same evaluation time point as the efficacy evaluation time point of the GS4774 clinical trial. As a result, it was confirmed that the amount of HBsAg reduction was greater in the 1/2a clinical trial than in the control group of the GS4774 phase 2 clinical trial.

Through the comparison with the results of the GS4774 clinical trial, it was confirmed that it is difficult to overcome immune tolerance in chronic hepatitis B patients only by taking oral antiviral agents, and that the combined administration of CVI-HBV-002 is more effective in HBeAg loss or HBeAg reduction.

### 1-3. Comparison of TAF phase 3 clinical trial results (1 year)

Second, the results of the phase 3 clinical trial (1 year) of TAF were compared with the results of the 1/2a clinical trial of CVI-HBV-002. In the phase 3 clinical trial of TAF, TDF was used as an active control group, and the detailed results of the clinical trial are the same as the results of the phase 3 clinical trial of Tenofoviralafenamide (TAF) described above in the description o the related art [Table 4]. The TAF phase 3 clinical trial results (including active control TDF results) were used to compare the 1/2a efficacy results of CVI-HBV-002 because the subjects were the most similar to the 1/2a clinical trial of CVI-HBV-002 among oral antiviral agent clinical trials, including patients with existing antiviral agent treatment experience in the TAF phase 3 clinical trial. In the phase 3 clinical trial of TAF, the proportion of subjects who had antiviral treatment experience was about 20% to 26%, although it varied slightly by group.

The results are shown in Figure 4 and Table 10.

Figure 4 is a graph showing the results of comparing the HBeAg loss rate in the CVI-HBV-002 1/2a clinical trial and the TAF phase 3 clinical trial.

**[Table 10]**

| Comparison of CVI-HBV-002 phase 1/2a and TAF phase 3 clinical trial results (1 year) | | | | | | | | | |
|---|---|---|---|---|---|---|---|---|---|
| Clinic al trial | | 1/2a clinical trial for CVI-HBV- 002 | | | Phase 3 clinical trial for TAF (control group TDF) | | | | |
| Main recrui tment subjec ts | Patients taking antiviral agents | | | Patients receiving antiviral agents for the first time and patients undergoing treatment | | | | | |
| Effica cy evalua tion point | HBsAg& T cell response: 4 weeks after last dose (W12,14,24) HBeAg: 12 weeks after last dose (W20,22,32) | | | 1 year (week 48) | | | | | |
| Drug | CVI-HBV-002 | | | TDF | | | TAF | | |
| Baseli ne HBeAg | HBeAg (+) (N=35 ) | HBeAg (-) (N=16 ) | Total (N=51 ) | HBeAg (+) (N=29 2) | HBeAg (-) (N=14 0) | Total (N=43 2) | HBeAg (+) (N=58 1) | HBeAg (-) (N=28 5) | Tot al (N= 866 ) |
| HBeAg loss n/N (%) | 8/35 (23%) | NA | 8/35 (23%) | 34/28 5 (12%) | NA | 34/28 5 (12%) | 78/56 5 (14%) | NA | 78/ 565 (14 %) |
| HBsAg loss n/N (%) | 0 | 0 | 0 | 1/288 (1%) | 0 | 1/428 (<1%) | 4/576 (1%) | 0 | 4/8 61 (<1 %) |
| HBV specif ic T cell respon se . IFN-γ spot formin g cells compar ed to before admini strati on > 1 | 26/32 (81.2 %) | 15/16 (93.8 %) | 41/48 (85.4 %) | No result | | | | | |

As shown in Figure 4 and Table 10, when comparing efficacy, CVI-HBV-002 showed the highest HBeAg loss rate of 23%, followed by TAF of 14% and TDF of 12%. Considering that the efficacy evaluation time points of the phase 1/2a clinical trial were week 20, week 22, and week 32 significantly ahead of the TAF clinical trial evaluation time point (Week 48, 1 year) compared to the baseline time point depending on the group, the above results were very encouraging and showed a synergistic effect of the combined administration of CVI-HBV-002. In addition, no subjects showed HBsAg loss in the CVI-HBV-002 group, the HBsAg loss rate was less than 1% in the TAF group, and only 1 out of 428 patients showed HBsAg loss in the TDF group. Meanwhile, HBV-specific T cell response was not included in the evaluation items in the oral antiviral agent clinical trial, so it could not be compared.

### Results of CVI-HBV-002 phase 1/2a long-term follow-up study

### 2-1. Efficacy results of 1/2a clinical trial long-term follow-up study

A long-term follow-up study was conducted to confirm the efficacy of the hepatitis B therapeutic vaccine (CVI-HBV-002) in subjects who participated in the phase 1/2a clinical trial, and a total of 24 patients participated in this study.

The results are shown in Table 11.

**[Table 11]**

| Summary of long-term follow-up efficacy evaluation results | | | | | |
|---|---|---|---|---|---|
| | Test item | Before vaccine administrati on | 4 weeks after last vaccine administrati on | Long-term follow -up | Note |
| S00 4 | HBsA g | 22, 684 | 18,344 | 128 | High HBsAg reducti on rate (99%) |
| | HBeA g | - | - | - | |
| | Anti -HBe | + | + | + | |
| S03 5 | HBsA g | 20,885 | 17,308 | 121 | High HBsAg reducti on rate (99%) |
| | HBeA g | + | + | + | |
| | Anti -HBe | - | - | - | |
| S00 5 | HBsA g | 1,544 | 1, 196 | 498 | HBeAg loss HBsAg reducti on rate (68%) |
| | HBeA g | + | + | - | |
| | Anti -HBe | - | - | - | |
| S02 6 | HBsA g | 5, 040 | 4,252 | 2, 802 | HBeAg loss HBsAg reducti on rate (45%) |
| | HBeA g | + | + | - | |
| | Anti -HBe | - | - | - | |

As shown in Table 11, as a result of quantitative analysis of HBsAg, it was confirmed that the HBsAg level was additionally reduced in 15 patients, 5 of which showed the HBsAg reduction rate of 50% or more, and 2 patients showed the HBsAg reduction rate of 99% or more. As a result of analyzing HBeAg serum loss, two additional patients showed HBeAg serum loss compared to the results at 12 weeks after the last CVI-HBV-002 administration in the 1/2a clinical trial.

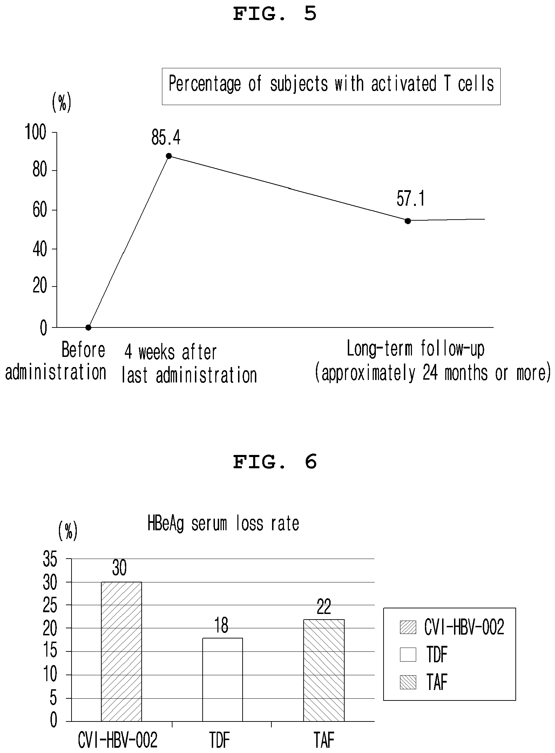

Next, when the HBV-specific T cell immune response was analyzed, 8 out of 14 subjects (57.1%) showed the formation of more IFN-γ spots compared to the 1/2a clinical trial baseline, confirming that T-cell activation was continuously maintained (see Figure 5). Among them, 6 (42.9%) patients showed the formation of more than twice as many IFN-γ spots as the baseline. Through these results, it was confirmed that the immune activation by the therapeutic vaccine CVI-HBV-002 continued for a long time, and the possibility as a treatment for chronic hepatitis B was confirmed again.

### 2-2. Comparison of CVI-HBV-002 long-term follow-up results and TAF phase 3 clinical trial results (2 years)

The results of a long-term follow-up study conducted about 4 years after the subjects enrolled in the 1/2a phase clinical trial were compared with the results of the second-year efficacy evaluation of the TAF phase 3 clinical trial. The results are shown in Figure 6 and Table 12.

Figure 6 is a graph showing the results of comparing the HBeAg loss rate in the CVI-HBV-002 long-term follow-up study and the TAF phase 3 clinical trial.

**[Table 12]**

| Comparison of CVI-HBV-002 long-term follow-up results and TAF phase 3 clinical trial results (2 years) | | | | | | | | | |
|---|---|---|---|---|---|---|---|---|---|
| Clinic al trial | Long-term follow-up study of phase 1/2a clinical trial | | | Phase 3 clinical trial for TAF (control group TDF) | | | | | |
| Effica cy evalua tion point | Average 4 years from phase 1/2a baseline | | | 2 years (week 96) | | | | | |
| Drug | CVI-HBV-002 | | | TDF | | | TAF | | |
| Baseli ne HBeAg | HBeAg (+) (N=20 ) | HBeA g (-) (N=4 ) | Tota l (N=2 4) | HBeA g (+) (N=2 92) | HBeA g (-) (N=1 40) | Tota l (N=4 32) | HBeA g (+) (N=5 81) | HBeA g (-) (N=2 85) | Tota l (N=8 66) |
| HBeAg loss n/N (%) | 6/20 (30%) | NA | 6/20 (30% ) | 51/2 85 (18% ) | NA | 51/2 85 (18% ) | 123/ 565 (22% ) | NA | 123/ 565 (22% ) |
| HBsAg loss n/N (%) | 0 | 0 | 0 | 4/28 8 (1%) | 0 | 4/42 8 (<1% ) | 7/57 6 (1%) | 1/28 1 (<1% ) | 8/86 1 (<1% ) |
| Averag e HBsAg reduct ion log₁₀IU /ml | -0.20 | - 0.60 * | - 0.27 * | - 0.64 | - 0.10 | No resu lt | - 0.51 | - 0.14 | No resu lt |
| HBV specif ic T cell respon se . IFN-γ spot formin g cells compar ed to before admini strati on > 1 | 5/10 (50%) | 3/4 (75% ) | 8/14 (57. 1%) | No result | | | | | |

| | | | | | | | | | |
|---|---|---|---|---|---|---|---|---|---|
| *Subjects with very high HBV DNA and HBsAg levels due to low medication compliance with antiviral agents were excluded. | | | | | | | | | |

As shown in Figure 6 and Table 12, as a result of comparing the results of the long-term follow-up study conducted after an average of 4 years after subjects enrolled in the phase 1/2a clinical trial and the second year efficacy evaluation result of the phase 3 clinical trial of TAF, the HBeAg loss rate after CVI-HBV-002 administration was still the highest.

Regarding the HBeAg loss rate, the HBeAg loss rate in the CVI-HBV-002 group was 30%, 22% in the TAF group, and 18% in the TDF group.

To compensate for the absence of a control group in the 1/2a clinical trial using CVI-HBV-002, other clinical trials that can be replaced with a possible control group were investigated and the efficacy was compared. As a result, it was confirmed that the combined administration of CVI-HBV-002 was more effective than the administration of antiviral drug alone. In the clinical trial of Gilead's therapeutic vaccine (GS4774), no patients showed HBeAg loss in the group adminisyered with an antiviral agent alone, and the degree of T cell activation was lower than that of the group administered with GS4774, whereas 23% of patients showed HBeAg loss, and 85.4% of patients showed T-cell activation after CVI-HBV-002 administration. These results confirm that CVI-HBV-002 overcomes immune tolerance in chronic hepatitis B patients.

In addition, when compared with the efficacy results of the phase 3 clinical trial of TAF, which also included patients taking antiviral agents, the HBeAg loss rate was the highest (23%), even though the efficacy evaluation time point was earlier after CVI-HBV-002 administration. From the results, it was confirmed that the combined administration of CVI-HBV-002 had a higher therapeutic effect compared to the administration of TAF(14%) or TDF(12%) alone. When the results of 2-year administration of oral antiviral agents and the long-term follow-up results of CVI-HBV-002 were compared, the HBeAg loss rate of CVI-HBV-002 was the highest at 30%. Of course, the 1/2a clinical trial using CVI-HBV-002 was initially conducted on a small scale, making it difficult to confirm HBsAg loss, but since HBeAg loss is preceded by HBsAg loss, the higher HBeAg loss rate than the oral antiviral agents is an encouraging result. These results support the possibility of a combination therapy that enables a rapid cure by administering CVI-HBV-002 in combination with an antiviral agent to give a synergistic effect to the antiviral agent's inhibitory effect on hepatitis B virus proliferation.

## Claims

1. A pharmaceutical combined formulation for use in the prevention or treatment of chronic hepatitis B comprising an antiviral agent for hepatitis B; and a vaccine composition including an antigen, a lipopeptide and a poly(I:C) adjuvant, wherein the antiviral agent for hepatitis B is an oral antiviral agent, wherein the oral antiviral agent is at least one selected from the group consisting of Entecavir, Tenofovirdisoproxil fumarate (TDF), Tenofoviralafenamide fumarate (TAF), Besifovirdipivoxil maleate, Lamivudine, Telbivudine, Clevudine and Adefovirdipivoxil, wherein the antigen is an entire surface antigen (L-HBsAg) of HBV (Hepatitis B Virus), and wherein the lipopeptide is at least one selected from the group consisting of Pam3Cys-SKKKK, PHC-SKKKK, Ole2PamCys-SKKKK, Pam2Cys-SKKKK, PamCys(Pam)-SKKKK, Ole2Cys-SKKKK, Myr2Cys-SKKKK, PamDhc-SKKKK, PamCSKKKK and Dhc-SKKKK.

2. The pharmaceutical combined formulation for use in the prevention or treatment of chronic hepatitis B according to claim 1, wherein the lipopeptide and the poly(I:C) are included in a weight ratio of 0.1 to 10:1.

3. The pharmaceutical combined formulation for use in the prevention or treatment of chronic hepatitis B according to claim 1, wherein the vaccine composition is an aqueous solution formulation.

4. The pharmaceutical combined formulation for use in the prevention or treatment of chronic hepatitis B according to claim 1, wherein the vaccine composition further includes at least one selected from the group consisting of pharmaceutically acceptable carriers, diluents and adjuvants.

5. The pharmaceutical combined formulation for use in the prevention or treatment of chronic hepatitis B according to claim 1, wherein the vaccine composition is administered through any one administration route selected from the group consisting of oral, transdermal, intramuscular, intraperitoneal, intravenous, subcutaneous and nasal administration.

6. A combined formulation kit for use in the prevention or treatment of chronic hepatitis B comprising a first component containing an antiviral agent for hepatitis B; and a second component containing a vaccine composition including an antigen, a lipopeptide and a poly(I:C) adjuvant, wherein the antiviral agent for hepatitis B is an oral antiviral agent, wherein the oral antiviral agent is at least one selected from the group consisting of Entecavir, Tenofovirdisoproxil fumarate (TDF), Tenofoviralafenamide fumarate (TAF), Besifovirdipivoxil maleate, Lamivudine, Telbivudine, Clevudine and Adefovirdipivoxil, wherein the antigen is an entire surface antigen (L-HBsAg) of HBV (Hepatitis B Virus), and wherein the lipopeptide is at least one selected from the group consisting of Pam3Cys-SKKKK, PHC-SKKKK, Ole2PamCys-SKKKK, Pam2Cys-SKKKK, PamCys(Pam)-SKKKK, Ole2Cys-SKKKK, Myr2Cys-SKKKK, PamDhc-SKKKK, PamCSKKKK and Dhc-SKKKK.

## Patentansprüche

1. Pharmazeutische Kombinationsformulierung zur Verwendung bei der Prävention oder Behandlung von chronischer Hepatitis B, umfassend ein antivirales Mittel gegen Hepatitis B und eine Impfstoffzusammensetzung, die ein Antigen, ein Lipopeptid und ein Poly(I:C)-Adjuvans enthält, wobei es sich bei dem antiviralen Mittel gegen Hepatitis B um ein orales antivirales Mittel handelt, wobei es sich bei dem oralen antiviralen Mittel um mindestens ein aus der aus Entecavir, Tenofovirdisoproxilfumarat (TDF), Tenofoviralafenamidfumarat (TAF), Besifovirdipivoxilmaleat, Lamivudin, Telbivudin, Clevudin und Adefovirdipivoxil bestehenden Gruppe ausgewähltes Mittel handelt, wobei es sich bei dem Antigen um ein vollständiges Oberflächenantigen (L-HBsAg) von HBV (Hepatitis-B-Virus) handelt und wobei es sich bei dem Lipopeptid um mindestens ein aus der aus Pam3Cys-SKKKK, PHC-SKKKK, Ole2PamCys-SKKKK, Pam2Cys-SKKKK, PamCys(Pam)-SKKKK, Ole2Cys-SKKKK, Myr2Cys-SKKKK, PamDhc-SKKKK, PamCSKKKK und Dhc-SKKKK bestehenden Gruppe ausgewähltes Lipopeptid handelt.

2. Pharmazeutische Kombinationsformulierung zur Verwendung bei der Prävention oder Behandlung von chronischer Hepatitis B nach Anspruch 1, wobei das Lipopeptid und das Poly(I:C) in einem Gewichtsverhältnis von 0,1 bis 10:1 enthalten sind.

3. Pharmazeutische Kombinationsformulierung zur Verwendung bei der Prävention oder Behandlung von chronischer Hepatitis B nach Anspruch 1, wobei es sich bei der Impfstoffzusammensetzung um eine wässrige Lösungsformulierung handelt.

4. Pharmazeutische Kombinationsformulierung zur Verwendung bei der Prävention oder Behandlung von chronischer Hepatitis B nach Anspruch 1, wobei die Impfstoffzusammensetzung weiterhin mindestens eine aus der aus pharmazeutisch unbedenklichen Trägern, Verdünnungsmitteln und Adjuvantien bestehenden Gruppe ausgewählte Komponente enthält.

5. Pharmazeutische Kombinationsformulierung zur Verwendung bei der Prävention oder Behandlung von chronischer Hepatitis B nach Anspruch 1, wobei die Impfstoffzusammensetzung über eine aus der aus oraler, transdermaler, intramuskulärer, intraperitonealer, intravenöser, subkutaner und nasaler Verabreichung bestehenden Gruppe ausgewählte Verabreichungsroute verabreicht wird.

6. Kombinationsformulierungskit zur Verwendung bei der Prävention oder Behandlung von chronischer Hepatitis B, umfassend eine erste Komponente, die ein antivirales Mittel gegen Hepatitis B enthält, und eine zweite Komponente, die eine Impfstoffzusammensetzung enthält, die ein Antigen, ein Lipopeptid und ein Poly(I:C)-Adjuvans enthält, wobei es sich bei dem antiviralen Mittel gegen Hepatitis B um ein orales antivirales Mittel handelt, wobei es sich bei dem oralen antiviralen Mittel um mindestens ein aus der aus Entecavir, Tenofovirdisoproxilfumarat (TDF), Tenofoviralafenamidfumarat (TAF), Besifovirdipivoxilmaleat, Lamivudin, Telbivudin, Clevudin und Adefovirdipivoxil bestehenden Gruppe ausgewähltes Mittel handelt, wobei es sich bei dem Antigen um ein vollständiges Oberflächenantigen (L-HBsAg) von HBV (Hepatitis-B-Virus) handelt und wobei es sich bei dem Lipopeptid um mindestens ein aus der aus Pam3Cys-SKKKK, PHC-SKKKK, Ole2PamCys-SKKKK, Pam2Cys-SKKKK, PamCys(Pam)-SKKKK, Ole2Cys-SKKKK, Myr2Cys-SKKKK, PamDhc-SKKKK, PamCSKKKK und Dhc-SKKKK bestehenden Gruppe ausgewähltes Lipopeptid handelt.

## Revendications

1. Formulation pharmaceutique combinée destinée à être utilisée dans la prévention ou le traitement de l'hépatite B chronique comprenant un agent antiviral pour l'hépatite B ; et une composition vaccinale comprenant un antigène, un lipopeptide et un adjuvant poly(I:C), l'agent antiviral pour l'hépatite B étant un agent antiviral oral, l'agent antiviral oral étant au moins un agent choisi dans le groupe constitué de l'entécavir, le fumarate de ténofovirdisoproxil (TDF), le fumarate de ténofoviralafénamide (TAF), le maléate de bésifovirdipivoxil, la lamivudine, la telbivudine, la clévudine et l'adéfovirdipivoxil, l'antigène étant un antigène de surface entier (L-HBsAg) du VHB (virus de l'hépatite B), et le lipopeptide étant au moins l'un choisi dans le groupe constitué de Pam3Cys-SKKKK, PHC-SKKKK, Ole2PamCys-SKKKK, Pam2Cys-SKKKK, PamCys(Pam)-SKKKK, Ole2Cys-SKKKK, Myr2Cys-SKKKK, PamDhc-SKKKK, PamCSKKKK et Dhc-SKKKK.

2. Formulation pharmaceutique combinée destinée à être utilisée dans la prévention ou le traitement de l'hépatite B chronique selon la revendication 1, le lipopeptide et le poly(I:C) étant inclus dans un rapport pondéral de 0,1 à 10:1.

3. Formulation pharmaceutique combinée destinée à être utilisée dans la prévention ou le traitement de l'hépatite B chronique selon la revendication 1, la composition vaccinale étant une formulation en solution aqueuse.

4. Formulation pharmaceutique combinée destinée à être utilisée dans la prévention ou le traitement de l'hépatite B chronique selon la revendication 1, la composition vaccinale comprenant en outre au moins l'un choisi dans le groupe constitué de véhicules, de diluants et d'adjuvants pharmaceutiquement acceptables.

5. Formulation pharmaceutique combinée destinée à être utilisée dans la prévention ou le traitement de l'hépatite B chronique selon la revendication 1, la composition vaccinale étant administrée par l'intermédiaire d'une voie d'administration choisie dans le groupe constitué de l'administration orale, transdermique, intramusculaire, intrapéritonéale, intraveineuse, sous-cutanée et nasale.

6. Kit de formulation combinée destiné à être utilisé dans la prévention ou le traitement de l'hépatite B chronique comprenant un premier composant contenant un agent antiviral pour l'hépatite B ; et un deuxième composant contenant une composition vaccinale comprenant un antigène, un lipopeptide et un adjuvant poly(I:C), l'agent antiviral pour l'hépatite B étant un agent antiviral oral, l'agent antiviral oral étant au moins un agent choisi dans le groupe constitué de l'entécavir, le fumarate de ténofovirdisoproxil (TDF), le fumarate de ténofoviralafénamide (TAF), le maléate de bésifovirdipivoxil, la lamivudine, la telbivudine, la clévudine et l'adéfovirdipivoxil, l'antigène étant un antigène de surface entier (L-HBsAg) du VHB (virus de l'hépatite B), et le lipopeptide étant au moins l'un choisi dans le groupe constitué de Pam3Cys-SKKKK, PHC-SKKKK, Ole2PamCys-SKKKK, Pam2Cys-SKKKK, PamCys(Pam)-SKKKK, Ole2Cys-SKKKK, Myr2Cys-SKKKK, PamDhc-SKKKK, PamCSKKKK et Dhc-SKKKK.
